# EUROPEAN PATENT APPLICATION

(11) **EP 4 647 118 A1**
(43) Date of publication of application: **12.11.2025**
(21) Application number: 24792799.9
(22) Date of filing: 02.02.2024
(51) Int. Cl.: A61N 1/40, A61N 1/08, A61F 7/00, A61B 18/00

(54) **COOLING SYSTEM FOR SKIN CARE DEVICE**

(30) Priority: 17.04.2023 KR 20230050347
(71) Applicant: Jeisys Medical Inc., Seoul 08501 (KR)
(72) Inventor: PARK, Jong Jin, Seoul 08501 (KR); PARK, Jong Seung, Seoul 08501 (KR)
(74) Representative: Dompatent Partnerschaft von Patent- und Rechtsanwälten mbB
(86) International application number: PCT/KR2024/001629
(87) International publication number: WO 2024/219611

(57) **Abstract**

The present disclosure relates to a cooling system for a skin care device including a storage container storing a cooling fluid; a chamber in which the cooling fluid transferred from the storage container is filled; a discharge valve for discharging or blocking the cooling fluid filled in the chamber; a fluid level sensor for measuring a level of the cooling fluid filled in the chamber; and a pressurizer for increasing an internal pressure of the storage container so that an internal pressure of the storage container becomes greater than an internal pressure of the chamber, based on a value of a water level measured by the fluid level sensor being maintained constantly for a preset period.

## Description

### [TECHNICAL FIELD]

The present disclosure relates to a cooling system for a skin care device.

### [BACKGROUND ART]

A skin care device that cleans the skin by removing wrinkles, restoring skin elasticity, and removing sebum is being developed. This is because cleanly managed skin makes people look younger and contributes to an attractive appearance.

The skin care device includes a method for transferring ultrasound to the skin (HIFU type), a method for transferring high frequency waves to the skin (RF type), and a method for transferring laser light to the skin (Optical type).

Here, the method for transferring high frequency waves to the skin is that high frequency waves generated from the RF electrode is applied to the target area of the skin through the epidermis of the skin. As a result, coagulation necrosis of the target area of the skin is induced, so that collagen and elastic fibers of the target area of the skin are removed, and new collagen and elastic fibers can be formed. In addition, coagulation necrosis of the target area of the skin is also effective in improving skin pigmentation, acne scars, and wrinkles.

In addition, the conventional skin care device has a problem in that when the high-frequency application process of the RF electrode continues, the temperature of the target area of the skin increases excessively, causing thermal burns to occur in the target area of the skin.

### [DETAILED DESCRIPTION OF THE INVENTION]

### [TECHINICAL PROBLEM]

The present disclosure is to provide a cooling system for a skin care device that can induce pain relief and skin soothing and prevent thermal injury to the skin by transferring a cooling fluid to a target area of the skin to which high frequency wave is applied.

In addition, the present disclosure is to provide a cooling system for a skin care device that can prevent a cooling fluid from remaining in a storage container by transferring all of the cooling fluid stored in a storage container to a chamber.

Technical problems of the inventive concept are not limited to the technical problems mentioned above, and other technical problems not mentioned will be clearly understood by those skilled in the art from the following description.

### [TECHNICAL SOLUTION]

In an aspect of the present disclosure, a cooling system for a skin care device may include a storage container storing a cooling fluid; a chamber in which the cooling fluid transferred from the storage container is filled; a discharge valve for discharging or blocking the cooling fluid filled in the chamber; a fluid level sensor for measuring a level of the cooling fluid filled in the chamber; and a pressurizer for increasing an internal pressure of the storage container so that an internal pressure of the storage container becomes greater than an internal pressure of the chamber, based on a value of a water level measured by the fluid level sensor being maintained constantly for a preset period.

Furthermore, based on the value of a water level measured by the fluid level sensor being maintained constantly, the pressurizer may include a pressurizing heater for heating the storage container to increase the internal pressure of the storage container.

Furthermore, after the pressurizing heater heats the storage container, based on the value of a water level measured by the fluid level sensor being maintained constantly, the pressurizing heater may stop heating the storage container.

Furthermore, the pressurizing heater may have a shape that surrounds the storage container.

Furthermore, the pressurizer may further include a heat insulating material that surrounds the storage container and the pressurizing heater.

Furthermore, the system may further include a storage container loading part in which the storage container is detachably loaded; and a connecting pipe connecting the storage container loading part and the chamber.

Furthermore, the system may further include a check valve provided in the connecting pipe to control a flow of the cooling fluid in the connecting pipe in a direction toward the chamber.

Furthermore, the system may further include a barb fitting part connecting the connecting pipe and the chamber, wherein the barb fitting part may include: a barb fitting body; a barb fitting coupling part provided on one side of the barb fitting body and detachably coupled to the chamber; and a barb fitting clamp provided on the other side of the barb fitting body and detachably coupled to the connecting pipe, wherein a plurality of barb protrusions is formed at an interval along a central axis of the barb fitting clamp on an outer surface of the barb fitting clamp.

Furthermore, the system may further include an auxiliary barb fitting part connecting the storage container and the connecting pipe, wherein the auxiliary barb fitting part may include: a barb fitting body; a barb fitting coupling part provided on one side of the barb fitting body and detachably coupled to the storage container; and a barb fitting clamp provided on the other side of the barb fitting body and detachably coupled to the connecting pipe, wherein a plurality of barb protrusions is formed at an interval along a central axis of the barb fitting clamp on an outer surface of the barb fitting clamp.

Furthermore, the barb protrusion may have a shape of which a diameter decreases as being away from the barb fitting body.

Furthermore, the system may further include a pressure maintaining part for maintaining the internal pressure of the chamber at a constant level.

Furthermore, the pressure maintaining part may include a pressure maintaining heater for heating the chamber at a constant time interval so that the internal pressure of the chamber is maintained at a constant level.

Other specific details of the present disclosure are included in the detailed description and drawings.

### [ADVANTAGEOUS EFFECTS OF THE INVENTION]

According to the present disclosure, the cooling system for a skin care device according to an embodiment of the present disclosure may induce pain relief and skin soothing by delivering a cooling fluid to a target area of the skin, and may have the effect of preventing thermal injury of the skin.

In addition, the cooling system for a skin care device according to an embodiment of the present disclosure has the effect of transferring all of the cooling fluid stored in the storage container 10 to the chamber 20.

The effects of the present disclosure are not limited to the effects mentioned above, and other effects not mentioned will be clearly understood by those skilled in the art from the description.

### [DESCRIPTION OF THE DRAWINGS]

FIG. 1 is a perspective view illustrating a cooling system for a skin care device according to an embodiment of the present disclosure.
FIG. 2 is an exploded perspective view illustrating a storage container and a storage container loading part of a cooling system for a skin care device according to one embodiment of the present disclosure.
FIG. 3 is a front view illustrating a cooling system for a skin care device according to an embodiment of the present disclosure.
FIGS. 4 to 6 are front views illustrating an operation process of a cooling system for a skin care device according to an embodiment of the present disclosure.
FIG. 7 is a perspective view illustrating a one-touch fitting part of a cooling system for a skin care device according to an embodiment of the present disclosure.
FIG. 8 is a perspective view illustrating the barb fitting part of a cooling system for a skin care device according to one embodiment of the present disclosure.

### [BEST MODE]

The advantages and features of the present disclosure, and methods for achieving them, will become clear with reference to the embodiments described in detail below along with the accompanying drawings. However, the present disclosure is not limited to the embodiments disclosed below and may be implemented in various different forms. The present embodiments are merely provided to ensure that the present disclosure is complete, and provided to fully convey the scope of the present disclosure to those skilled in the art to which the present disclosure pertains. The present disclosure is defined only by the scope of the claims.

The terminology used herein is for the purpose of describing embodiments and is not intended to limit the disclosure. As used herein, singular forms also include plural forms, unless specifically stated otherwise in the context. As used in the specification, "comprises" and/or "comprising" does not exclude the presence or addition of one or more other elements in addition to the mentioned elements.

Unless otherwise defined, all terms (including technical and scientific terms) used in this specification may be used with meanings commonly understood by those skilled in the art to which this disclosure pertains. Additionally, terms defined in commonly used dictionaries are not to be interpreted ideally or excessively unless clearly specifically defined.

Hereinafter, the embodiments of the present disclosure will be described in detail with reference to the attached drawings.

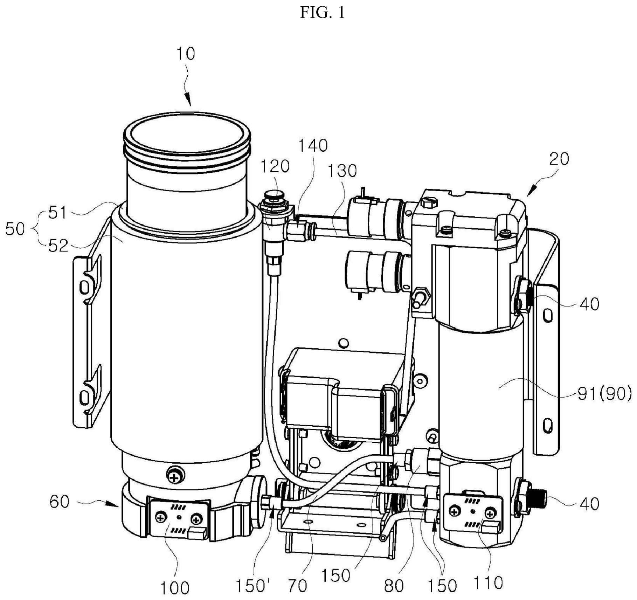

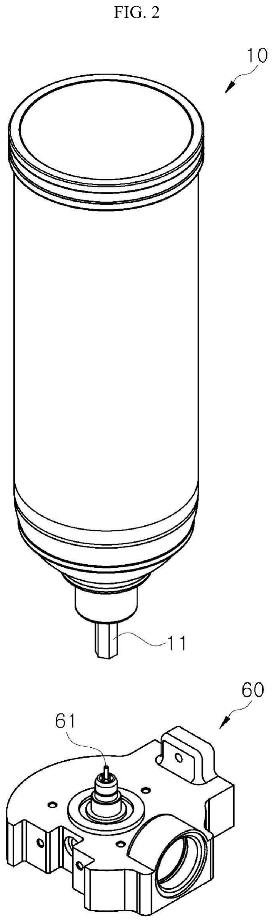

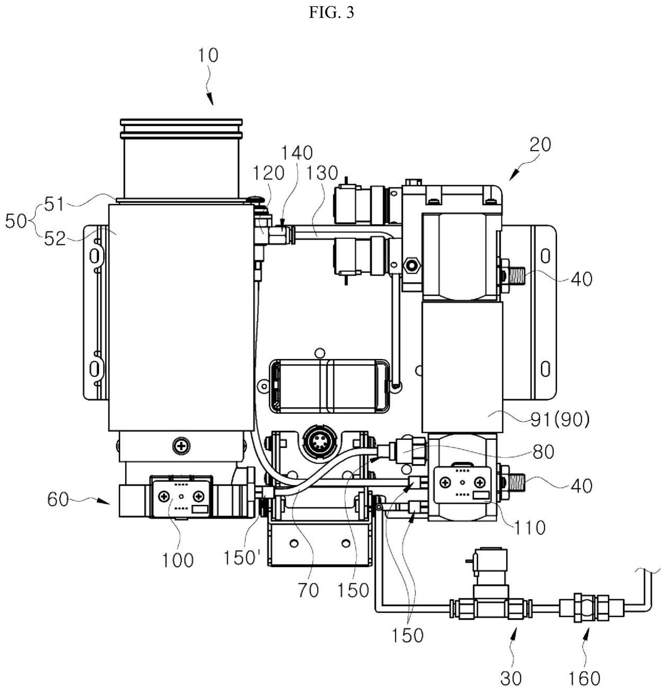

FIG. 1 is a perspective view illustrating a cooling system for a skin care device according to an embodiment of the present disclosure, FIG. 2 is an exploded perspective view illustrating a storage container 10 and a storage container loading part 60 of a cooling system for a skin care device according to one embodiment of the present disclosure, and FIG. 3 is a front view illustrating a cooling system for a skin care device according to an embodiment of the present disclosure.

As shown in FIG. 1, a cooling system for a skin care device according to an embodiment of the present disclosure includes a storage container 10, a chamber 20, a discharge valve 30, a fluid level sensor 40, and a pressurizer 50.

A cooling fluid is stored in the storage container. The cooling fluid stored in the storage container 10 may be supplied to the chamber 20. Furthermore, the cooling fluid may be a cooling gas or a cooling liquid. For example, HFO (hydrofluoro-olefin) may be used as the cooling gas, and water may be used as the cooling liquid.

As shown in FIG. 2, the storage container 10 may be formed in an overall cylindrical shape. In addition, a storage container discharging part 11 for discharging the cooling fluid may be provided on one side of the storage container 10. The storage container discharging part 11 may have a shape in which a diameter decreases as being away from the storage container 10. Meanwhile, the storage container 10 may be detachably coupled to a storage container loading part 60 to be described below. Accordingly, when the cooling fluid stored in the storage container 10 is completely consumed, the storage container 10 may be removed from the storage container loading part 60, and then a new storage container 10 may be coupled to the storage container loading part 60. At this time, the new storage container 10 may be filled with the cooling fluid inside.

The chamber 20 may be filled with the cooling fluid transferred from the storage container 10. The cooling fluid filled in the chamber 20 may be discharged to the outside through the discharge valve 30. For example, the chamber 20 may be formed in an overall longitudinal direction.

As shown in FIG. 3, the discharge valve 30 serves to discharge or block the cooling fluid filled in the chamber 20. For example, a mouse valve or a solenoid valve may be used as the discharge valve 30.

For example, the discharge valve 30 may connect the chamber 20 and the skin care device. At this time, the cooling fluid discharged through the discharge valve 30 may be delivered to the skin care device. As a result, the failure of the skin care device due to a temperature increase may be prevented by the cooling fluid delivered to the skin care device. Here, the discharge valve 30 may be connected to the skin care device through a coupler 160.

As another example, the discharge valve 30 may be positioned so that one side is connected to the chamber 20 and the other side is adjacent to a target area of the skin to which electric energy is applied by the skin care device. At this time, the cooling fluid discharged through the discharge valve 30 may be applied to the target area of the skin to which electric energy is applied by the skin care device. As a result, the cooling fluid applied to the target area of the skin to which electric energy is applied by the skin care device may induce pain relief and soothing of the target area of the skin.

As an example, the discharge valve 30 is opened and closed by the control of a controller (not shown) and may discharge or block the cooling fluid filled in the chamber 20. Here, the controller may be a microcomputer.

The fluid level sensor 40 may measure a water level of the cooling fluid filled in the chamber 20. For example, the fluid level sensor 40 may measure a pressure at a lower end of the chamber 20 and a pressure at an upper end of the chamber 20, respectively, and measure the differential pressure between the pressure at the lower end of the chamber 20 and the pressure at the upper end of the chamber 20, thereby calculating the water level of the chamber 20. Specifically, as the lower end of the chamber 20 is filled with the cooling fluid and the upper end of the chamber 20 is filled with air in the atmosphere, the fluid level sensor 40 may measure the pressure of the cooling fluid filled in the lower end of the chamber 20 and the pressure of the air in the atmosphere filled in the upper end of the chamber 20, respectively, and measure the differential pressure between the pressure of the cooling fluid in the chamber 20 and the pressure in the atmosphere, thereby calculating the water level of the chamber 20. As another example, the fluid level sensor 40 is provided at a reference water level point between the lower part of the chamber 20 and the upper part of the chamber 20, so that when the water level of the cooling fluid filled in the chamber 20 exceeds the reference water level point of the chamber 20, the fluid level sensor 40 may sense that the cooling fluid filled in the chamber 20 exceeds the fluid level sensor 40.

Meanwhile, when the value of the water level measured by the fluid level sensor 40 is maintained constant for a preset period, the cooling fluid stored in the storage container 10 may not be transferred to the chamber 20 because the internal pressure of the storage container 10 is lower than the internal pressure of the chamber 20. Therefore, it is necessary to increase the internal pressure of the storage container 10 so that the cooling fluid stored in the storage container 10 is transferred to the chamber 20 without any residue. For example, the set time may be 10 to 30 seconds.

The pressurizer 50 may increase the internal pressure of the storage container 10 so that the internal pressure of the storage container 10 becomes greater than the internal pressure of the chamber 20 when a value of water level measured by the fluid level sensor 40 is maintained constantly for a preset period. As a result, the cooling fluid stored in the storage container 10 may be transferred to the chamber 20.

The pressurizer 50 may include a pressurizing heater 51, a heat insulating material 52, and a pressurizing temperature sensor (not shown).

The pressurizing heater 51 may heat the storage container 10 to increase the internal pressure of the storage container 10 when the value of the water level measured by the fluid level sensor 40 is maintained constantly. For example, the pressurizing heater 51 may have a form that surrounds the storage container 10. Specifically, the pressurizing heater 51 may have a ring shape.

For example, the pressurizing heater 51 may control the temperature of the storage container 10 to the room temperature to 50° C. For example, the room temperature may be 1° C to 30° C, but the present disclosure is not limited thereto.

The heat insulating material 52 surrounds the storage container 10 and the pressurizing heater 51 and may serve to insulate the storage container 10 and the pressurizing heater 51. Therefore, after the temperature of the storage container 10 increases due to heating by the pressurizing heater 51, the heat loss and pressure loss of the storage container 10 may be reduced by the heat insulating material 52.

The pressurizing temperature sensor may serve to measure the internal temperature of the storage container 10. The pressurizing temperature sensor may transmit the measured value of the internal temperature of the storage container 10 to the pressurizing heater 51. Thereafter, the pressurizing heater 51 may heat the storage container 10 so that the internal temperature of the storage container 10 increases based on the measured value of the internal temperature of the storage container 10 transmitted from the pressurizing temperature sensor.

For example, when the cooling fluid filled in the chamber 20 is discharged, in the case that the value of the water level measured by the fluid level sensor 40 remains constant or rises, the cooling fluid stored in the storage container 10 may be transferred to the chamber 20. At this time, the controller may determine that the remaining amount of the cooling fluid stored in the storage container 10 is sufficient.

In another example, when the cooling fluid filled in the chamber 20 is discharged, in the case that the value of the water level measured by the fluid level sensor 40 decreases, the cooling fluid stored in the storage container 10 may be in a state where the cooling fluid stored in the storage container 10 is not transferred to the chamber 20 because the amount of cooling fluid stored in the storage container 10 is small or absent. At this time, the controller may determine that the remaining amount of the cooling fluid stored in the storage container 10 is insufficient, and may emit an alarm sound indicating that the remaining amount of the storage container 10 needs to be checked.

The cooling system for a skin care device according to an embodiment of the present disclosure may further include a storage container loading part 60, a connecting pipe 70, a check valve 80, a pressure maintaining part 90, a pressure sensor 100 for the storage container, and a pressure sensor 110 for the chamber.

The storage container loading part 60 may be loaded with the storage container 10 in a detachable manner. As shown in FIG. 2, a storage container connection part 61 inserted into the storage container discharging part 11 of the storage container 10 may be formed on one side of the storage container loading part 60. The storage container connection part 61 has a smaller diameter than the storage container discharging part 11 and may be inserted into the storage container discharging part 11. In addition, a pipe connection part connecting the storage container 10 insertion part and the connecting pipe 70 may be formed on the other side of the storage container loading part 60.

The connecting pipe 70 may play a role in connecting the storage container loading part 60 and the chamber 20.

The check valve 80 is provided in the connecting pipe 70 and may serve to control the flow of the cooling fluid in the connecting pipe 70 toward the chamber 20.

The pressure maintaining part 90 is provided in the chamber 20 and serves to maintain the internal pressure of the chamber 20 at a constant level. Therefore, even in the case that the pressurizer 50 does not excessively increase the internal pressure of the chamber 20, the cooling fluid stored in the storage container 10 may be easily transferred to the chamber 20 as the internal pressure of the storage container 10 becomes greater than the internal pressure of the chamber 20.

The pressure maintaining part 90 may include a pressure maintaining heater 91 and a pressure maintaining temperature sensor.

The pressure maintaining heater 91 may heat the chamber 20 at a constant time interval so that the internal pressure of the chamber 20 is maintained at a constant level. Specifically, the pressure maintaining heater 91 may heat the chamber 20 at a constant time interval so that the internal temperature of the chamber 20 is maintained at a temperature higher than the room temperature.

As an example, the pressure maintaining heater 91 may control the temperature of the chamber 20 to the room temperature to 35° C. For example, the room temperature may be 1° C to 30° C, but the present disclosure is not limited thereto.

The pressure maintaining temperature sensor may measure the internal temperature of the chamber 20. The pressure maintaining temperature sensor may transmit the measured value of the internal temperature of the chamber 20 to the pressure maintaining heater 91. Thereafter, the pressure maintaining heater 91 may heat the chamber 20 at a constant time interval so that the internal temperature of the chamber 20 is maintained constantly based on the measurement value of the internal temperature of the storage container 10 transmitted from the pressurizing temperature sensor.

The pressure sensor 100 for the storage container may measure the internal pressure of the storage container 10, and the pressure sensor 110 for the chamber may measure the internal pressure of the chamber 20. Here, the measurement value of the pressure sensor 100 for the storage container measuring the internal pressure of the storage container 10 and the measurement value of the pressure sensor 110 for the chamber measuring the internal pressure of the chamber 20 may be transmitted to the controller, respectively. Thereafter, the controller may control the pressurizer 50 to increase the internal pressure of the storage container 10 when the measured value of the internal pressure of the storage container 10 transmitted from the pressure sensor 100 for the storage container is equal to or smaller than the measured value of the internal pressure of the chamber 20 transmitted from the pressure sensor 110 for the chamber.

The cooling system for a skin care device according to an embodiment of the present disclosure may further include a relief valve 120.

The relief valve 120 may be opened and closed by the user's manipulation to discharge or stop the cooling fluid filled in the chamber 20 to the outside.

As an example, the relief valve 120 may discharge the cooling fluid filled in the chamber 20 to the outside through a relief pipe 130. The relief pipe 130 may be connected to the relief valve 120.

Hereinafter, the operation process of the cooling system for a skin care device according to an embodiment of the present disclosure will be described.

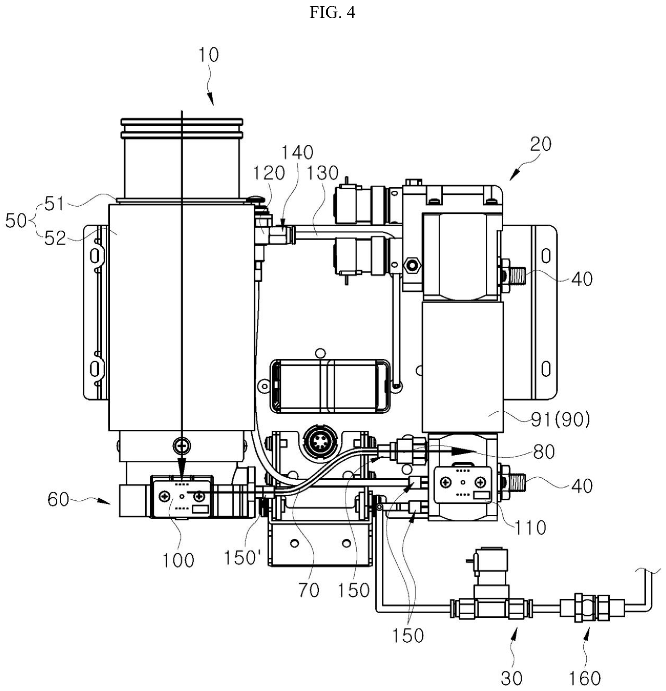

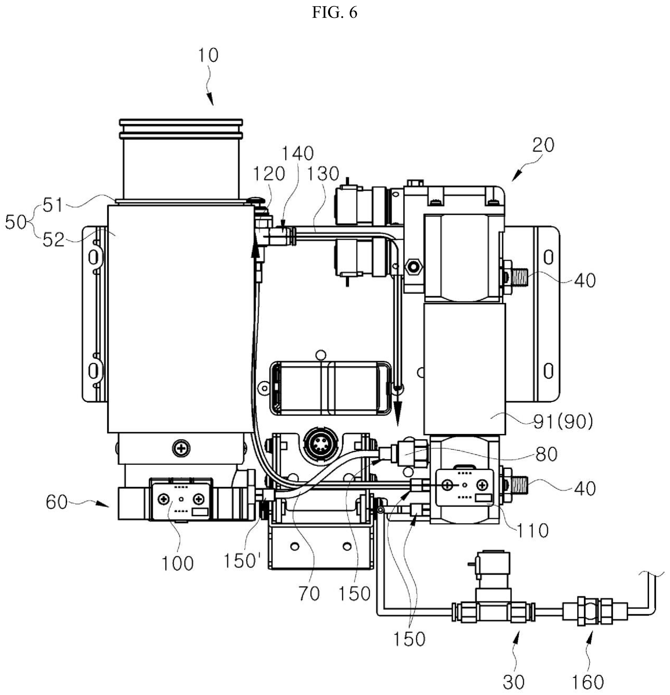

FIGS. 4 to 6 are front views illustrating an operation process of the cooling system for a skin care device according to an embodiment of the present disclosure. The following process may be performed by the controller.

First, the cooling fluid stored in the storage container 10 is transferred to the chamber 20 (See FIG. 4.).

Thereafter, the fluid level sensor 40 measures the water level of the cooling fluid filled in the chamber 20. At this time, in the case that the value of the water level of the cooling fluid filled in the chamber 20 measured by the fluid level sensor 40 is maintained constantly, the cooling fluid stored in the storage container 10 may not be transferred to the chamber 20 through the connecting pipe 70 and the check valve 80. In this case, the pressurizer 50 increases the internal pressure of the storage container 10 so that the internal pressure of the storage container 10 becomes greater than the internal pressure of the chamber 20. As a result, the cooling fluid stored in the storage container 10 may be transferred to the chamber 20 through the connecting pipe 70 and the check valve 80 without any residue.

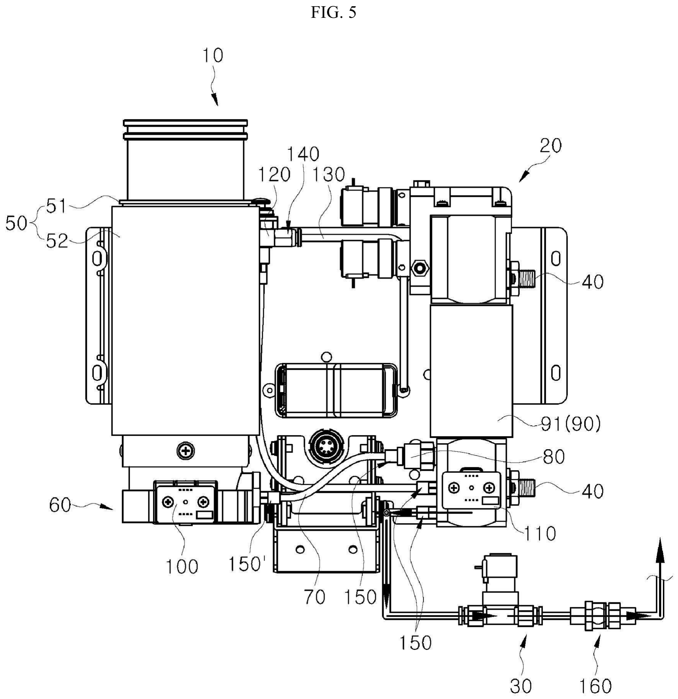

Next, the discharge valve 30 is opened by the control of the controller so that the cooling fluid filled in the chamber 20 may be discharged to the target through the discharge valve 30 (See FIG. 5.).

At this time, when the cooling fluid filled in the chamber 20 is discharged, in the case that the value of the water level measured by the fluid level sensor 40 is maintained constant or rises, the cooling fluid stored in the storage container 10 may be transferred to the chamber 20. In this case, the controller may determine that the remaining amount of the cooling fluid stored in the storage container 10 is sufficient.

In addition, when the cooling fluid filled in the chamber 20 is discharged, in the case that the value of the water level measured by the fluid level sensor 40 decreases, the cooling fluid stored in the storage container 10 may not be transferred to the chamber 20 because the cooling fluid stored in the storage container 10 is small or absent. At this time, the controller may determine that the remaining amount of the cooling fluid stored in the storage container 10 is insufficient, and may emit an alarm sound indicating that the remaining amount of the storage container 10 needs to be checked.

Here, the relief valve 120 may be forcibly opened by the user's operation to discharge the cooling fluid filled in the chamber 20 to the outside.

Meanwhile, when the cooling fluid filled in the chamber 20 is completely discharged for moving the cooling system for a skin care device or for repairing a malfunction, there is no need to use a barb fitting part 150 described below to prevent leakage between the relief valve 120 and the relief pipe 130 as the cooling fluid filled in the chamber 20 is completely discharged through the relief valve 120. Accordingly, the relief valve 120 and the relief pipe 130 may be easily detachably connected through a one-touch fitting part 140.

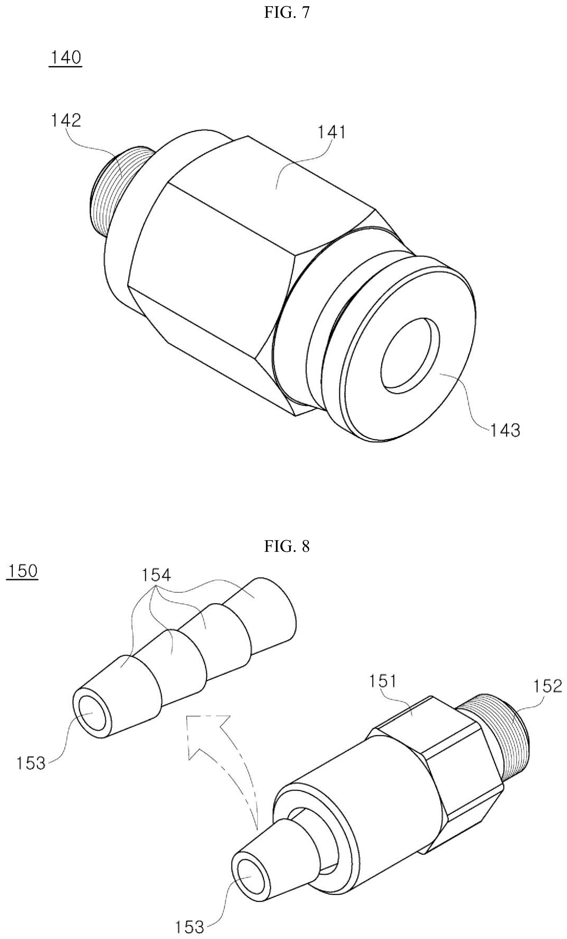

FIG. 7 is a perspective view illustrating a one-touch fitting part 140 of a cooling system for a skin care device according to an embodiment of the present disclosure.

As shown in FIG. 1 and FIG. 7, the cooling system for a skin care device according to an embodiment of the present disclosure may further include the one-touch fitting part 140.

The one-touch fitting part 140 may serve to detachably connect the relief valve 120 and the relief pipe 130. A flow path connecting the relief valve 120 and the relief pipe 130 may be formed inside the one-touch fitting part 140.

The one-touch fitting part 140 may include a one-touch fitting body 141, a one-touch fitting coupling part 142, and a one-touch fitting holder 143.

The one-touch fitting body 141 is a main body of the one-touch fitting part 140 and serves to connect the one-touch fitting connecting part 142 and the one-touch fitting holder 143.

The one-touch fitting connecting part 142 is provided on one side of the one-touch fitting body 141 and may be detachably connected to the relief valve 120. For example, the one-touch fitting connecting part 142 may have a bolt shape and may be screw-connected to the relief valve 120.

The one-touch fitting holder 143 is provided on the other side of the one-touch fitting body 141 and the relief pipe 130 may be detachably and easily inserted. Therefore, since the relief valve 120 and the relief pipe 130 are easily connected by the one-touch fitting part 140, the time required for the worker's assembling process and separation and fixing of the relief valve 120 and the relief pipe 130 may be reduced, thereby improving the worker's work efficiency.

For example, the one-touch fitting holder 143 may have a tube shape.

FIG. 8 is a perspective view illustrating the barb fitting part 150 of a cooling system for a skin care device according to one embodiment of the present disclosure.

As shown in FIG. 8, the cooling system for a skin care device according to an embodiment of the present disclosure may further include the barb fitting part 150 and an auxiliary barb fitting part 150.

The barb fitting part 150 may play a role in connecting the connecting pipe 70 and the chamber 20. A flow path connecting the connecting pipe 70 and the chamber 20 may be formed inside the barb fitting part 150.

The barb fitting part 150 may include a barb fitting body 151, a barb fitting coupling part 152, and a barb fitting clamp 153.

The barb fitting body 151 is a main body of the barb fitting part 150 and serves to connect the barb fitting coupling part 152 and the barb fitting clamp 153.

The barb fitting coupling part 152 may be provided on one side of the barb fitting body 151 and may be detachably coupled to the chamber 20. For example, the barb fitting coupling part 152 may have a bolt shape and may be screw-coupled to the chamber 20.

The barb fitting clamp 153 is provided on the other side of the barb fitting body 151 and may be detachably inserted into the connecting pipe 70. On the outer surface of the barb fitting clamp 153, a plurality of barb projections 154 may be formed at an interval along a central axis of the barb fitting body 151. The barb projection 154 may have a shape of which diameter decreases as being away from the barb fitting body 151. In other words, the barb projection 154 may have a truncated cone shape. Accordingly, the connecting pipe 70 inserted into the inside of the barb fitting clamp 153 may have improved sealing with the barb fitting clamp 153 by the plurality of barb projections 154. In this way, since the barb fitting part 150 has a relatively improved sealing property than the one-touch fitting part 140, it may prevent leakage of the cooling fluid at the connection part between the connecting pipe 70 and the check valve 80.

Meanwhile, the barb fitting part 150 is provided at the connection part between the relief valve 120 and the chamber 20, so that leakage of the cooling fluid may be prevented. In addition, the barb fitting part 150 is also provided at the connection part between the discharge valve 30 and the chamber 20, so that leakage of the cooling fluid may be prevented.

The auxiliary barb fitting part 150' may also play a role in connecting the connecting pipe 70 and the storage container 10. A flow path connecting the connecting pipe 70 and the storage container 10 may be formed inside this auxiliary barb fitting part 150'.

In addition, the auxiliary barb fitting part 150' may include the barb fitting body 151, the barb fitting coupling part 152, and the barb fitting clamp 153. The auxiliary barb fitting part 150' is the same as the barb fitting part 150 except that the barb fitting coupling part 152 is detachably coupled to the storage container 10, and the barb fitting clamp 153 is detachably coupled to the connecting pipe 70. Therefore, a detailed description thereof will be omitted.

Meanwhile, a skin care device according to an embodiment of the present disclosure may receive the cooling fluid discharged from the cooling system for the skin care device according to an embodiment of the present disclosure.

According to the present disclosure, the cooling system for a skin care device according to an embodiment of the present disclosure may induce pain relief and skin soothing by delivering a cooling fluid to a target area of the skin, and may have the effect of preventing thermal injury of the skin.

In addition, the cooling system for a skin care device according to an embodiment of the present disclosure has the effect of transferring all of the cooling fluid stored in the storage container 10 to the chamber 20.

Although the embodiments of the present disclosure have been described above with reference to the attached drawings, those skilled in the art will understand that the present disclosure may be implemented in other specific forms without changing the technical idea or essential features thereof. Therefore, it should be understood that the embodiments described above are exemplary in all respects and not restrictive.

## Claims

1. A cooling system for a skin care device, comprising:
a storage container storing a cooling fluid;
a chamber in which the cooling fluid transferred from the storage container is filled;
a discharge valve for discharging or blocking the cooling fluid filled in the chamber;
a fluid level sensor for measuring a level of the cooling fluid filled in the chamber; and
a pressurizer for increasing an internal pressure of the storage container so that an internal pressure of the storage container becomes greater than an internal pressure of the chamber, based on a value of a water level measured by the fluid level sensor being maintained constantly for a preset period.

2. The cooling system according to claim 1,
based on the value of a water level measured by the fluid level sensor being maintained constantly,
wherein the pressurizer includes a pressurizing heater for heating the storage container to increase the internal pressure of the storage container.

3. The cooling system according to claim 2,
after the pressurizing heater heats the storage container, based on the value of a water level measured by the fluid level sensor being maintained constantly,
wherein the pressurizing heater stops heating the storage container.

4. The cooling system according to claim 2,
wherein the pressurizing heater has a shape that surrounds the storage container.

5. The cooling system according to claim 2,
wherein the pressurizer further includes a heat insulating material that surrounds the storage container and the pressurizing heater.

6. The cooling system according to claim 1, further comprising:
a storage container loading part in which the storage container is detachably loaded; and
a connecting pipe connecting the storage container loading part and the chamber.

7. The cooling system according to claim 6, further comprising:
a check valve provided in the connecting pipe to control a flow of the cooling fluid in the connecting pipe in a direction toward the chamber.

8. The cooling system according to claim 6, further comprising:
a barb fitting part connecting the connecting pipe and the chamber,
wherein the barb fitting part includes:
a barb fitting body;
a barb fitting coupling part provided on one side of the barb fitting body and detachably coupled to the chamber; and
a barb fitting clamp provided on the other side of the barb fitting body and detachably coupled to the connecting pipe,
wherein a plurality of barb protrusions is formed at an interval along a central axis of the barb fitting clamp on an outer surface of the barb fitting clamp.

9. The cooling system according to claim 6, further comprising:
an auxiliary barb fitting part connecting the storage container and the connecting pipe,
wherein the auxiliary barb fitting part includes:
a barb fitting body;
a barb fitting coupling part provided on one side of the barb fitting body and detachably coupled to the storage container; and
a barb fitting clamp provided on the other side of the barb fitting body and detachably coupled to the connecting pipe,
wherein a plurality of barb protrusions is formed at an interval along a central axis of the barb fitting clamp on an outer surface of the barb fitting clamp.

10. The cooling system according to claim 8 or claim 9,
wherein the barb protrusion has a shape of which a diameter decreases as being away from the barb fitting body.

11. The cooling system according to claim 1, further comprising:
a pressure maintaining part for maintaining the internal pressure of the chamber at a constant level.

12. The cooling system according to claim 11,
wherein the pressure maintaining part includes a pressure maintaining heater for heating the chamber at a constant time interval so that the internal pressure of the chamber is maintained at a constant level.

13. A skin care device, which is supplied with the cooling fluid discharged from the cooling system for a skin care device according to any one of claims 1 to 12.
